(19) Europäisches Patentamt
European Patent Office
Office européen des brevets

(11) Publication number: 0 352 718
A2

(12) **EUROPEAN PATENT APPLICATION**

(21) Application number: 89113654.1

(22) Date of filing: 25.07.89

(51) Int. Cl.4: C07C 255/10 , C07C 55/32 , C07C 69/63 , C07C 211/15 , C07D 317/42 , C08G 63/68 , C08G 69/42 , C08G 73/10

(30) Priority: 25.07.88 US 223867

(43) Date of publication of application: 31.01.90 Bulletin 90/05

(84) Designated Contracting States: BE DE FR GB IT NL

(71) Applicant: E.I. DU PONT DE NEMOURS AND COMPANY
1007 Market Street
Wilmington Delaware 19898(US)

(72) Inventor: Anton, Douglas Robert
15 Mount Vernon Drive
Claymont Delaware 19703(US)
Inventor: Mckinney, Ronald James
1243 Lakewood Drive
Wilmington Delaware 19803(US)

(74) Representative: Abitz, Walter, Dr.-Ing. et al
Abitz, Morf, Gritschneder P.O. Box 86 01 09
D-8000 München 86(DE)

(54) **Partially fluorinated compounds and polymers.**

(57) Partially fluorinated compounds of specified formulas that are useful as chemical intermediates and monomers are disclosed. Partially fluorinated polymers prepared from one of these compounds are also disclosed.

EP 0 352 718 A2

# Partially Fluorinated Compounds and Polymers

## BACKGROUND OF THE INVENTION

### Field of the Invention

The present invention concerns partially fluorinated compounds that are useful as chemical intermediates or monomers. The invention further concerns partially fluorinated condensation polymers.

## SUMMARY OF THE BACKGROUND

Fluorinated intermediates are employed in the synthesis of a variety of chemical compounds, such as partially fluorinated polymers. Novel fluorinated intermediates and polymers derived therefrom are of significant interest to the chemical industry.

Partially fluorinated condensation polymers are known in the literature, see, e.g., I. L. Knunyants, C. Li & V. V. Shokina, Russ. Chem. Rev. 32:461 (1963) and the references described therein. These polymers generally have been made to obtain materials with improved thermo-oxidative stability and solvent resistance, as compared to nonfluorinated polymers.

Many partially fluorinated condensation polymers have been made with perfluoro diacids, $HOOC-(CF_2)_n-COOH$, as one component of the polymer, see, e.g., B. S. Marks & G. C. Schweiker, J. Polymer Sci. 43:229 (1960) wherein the synthesis of partially fluorinated polyamides containing perfluoro diacid units is disclosed. The polymers made from such perfluoro diacids, however, suffer from problems associated with the extreme hydrolytic instability of the perfluoroacyl functional groups. The strongly electron-withdrawing perfluoroalkyl group situated next to the carbonyl of the perfluoroacyl group activates the carbonyl to hydrolysis. It has been found that many of the resulting polymers hydrolyze in moist air.

P. Johncock, S. P. Barnett & P. A. Rickard, J. Polymer Sci./Polymer Chem. 24:2033 (1986) disclose the synthesis of a diacid wherein the fluoroalkyl chain is separated from the acyl group by a methylene spacer, $HOOC-CH_2-(CF_2)_3-CH_2-COOH$. Although polyesters made with this diacid are more hydrolytically stable than those made from perfluoro diacids, these compositions still hydrolyze under mild conditions. This acid also suffers from the ready loss of two moles of hydrogen fluoride from the molecule.

P. L. Coe, N. E. Milner & J. A. Smith, J. Chem. Soc./Perkin I 654 (1975) disclose use of perfluoroalkylcopper compounds to form certain polyfluoroalkyl-substituted acids and alcohols. These compounds are said to be of practical interest as surfactants. In one reaction, (1E,6E)-3,3,4,4,5,5-hexafluoro-1,7-diiodohepta-1,6-diene was reacted with copper(I) cyanide. Hydrolysis of the resulting unsaturated dinitrile yielded a diacid that was hydrogenated to give $HOOC-CH_2CH_2-(CF_2)_3-CH_2CH_2-COOH$.

U.S. 3,496,215 discloses hydrocyanation of unsaturated compounds using as a catalyst a compound of the structure $Ni(PXYZ)_4$ wherein X is OR; Y and Z are R or OR; and R is an alkyl or aryl radical of up to 18 carbon atoms. U.S. 3,496,217 discloses hydrocyanation of nonconjugated ethylenically unsaturated organic compounds using certain nickel complexes as a catalyst and certain zinc containing compounds as promoters. U.S. 3,496,218 discloses hydrocyanation of nonconjugated ethylenically unsaturated organic compounds using certain nickel complexes as catalysts and certain organoboron compounds as promoters.

C. A. Tolman, et al., Adv. in Catalysis 33:1 (1985) disclose homogeneous nickel-catalyzed olefin hydrocyanation, as well as unpromoted hydrocyanations of monoolefins and hydrocyanations promoted with Lewis acids.

## SUMMARY OF THE INVENTION

The present invention provides compounds of the formula

$H_2C=CH-R_f-CH_2\ CH_2-Y$ (1)

wherein Y is selected from the group consisting of -CN, $-CH_2NH_2$, -COOH, $-CH_2OH$, $-CH_2N=C=O$, $-CONH_2$, -CHO, -COCl, and -COOR, wherein R is $C_nH_{2n+1}$ and n is an integer from 1 to 10, inclusive; and

$R_f$ is a perfluorinated divalent organic radical selected from the group consisting of linear, branched, carbocyclic, and mixtures thereof having from 1 to about 40 carbon atoms and containing zero to about 20 ether linkages.

The invention additionally provides compounds of the formula

$Y'-CH_2CH_2-R_f-CH_2CH_2-Y'$ (2)

wherein $Y'$ is independently selected from the group consisting of -CN, $-CH_2NH_2$, $-CH_2OH$, $-CH_2N=C=O$, $-CONH_2$, -CHO, -COCl, and -COOR, wherein R is $C_nH_{2n+1}$ and n is an integer from 1 to 10, inclusive; and $R_f$ is as defined above.

Compound (2) can be employed in a condensation type of polymerization reaction to prepare polymers (A) and (B) of this invention. Polymer (A) can be either polymers comprising 100 mole percent of at least one independently selected repeating unit of the formula

$\{X-CH_2CH_2-R_f-CH_2CH_2-X-X'-Z-X'\}$ (3)

wherein X is independently selected from the group consisting of $NHCH_2-$, $OCH_2-$, -C(=O), and $-CH_2NH-(C=O)$; $X'$ is independently selected from the group consisting of NH-, O-, and -C(=O), provided that when X is $NHCH_2-$ or $OCH_2-$, $X'$ must be -C(=O), and also provided that when X is -C(=O) or $-CH_2NH(C=O)$, $X'$ must be NH- or O-; Z is any divalent organic radical; and $R_f$ is as defined above; or copolymers comprising from 1 to 99 mole percent of at least one independently selected repeating unit of formula (3) and from 99 to 1 mole percent of at least one independently selected repeating unit of the formula

$\{X''-Z-X''-X'-Z-X'\}$ (4)

wherein $X'$ and Z are as defined above, and Z is independently selected, and $X''$ is independently selected from the same group as $X'$, provided that when $X'$ is -C(=O), $X''$ must be NH- or O-, and also provided that when $X'$ is NH- or O-, $X''$ must be -C(=O). In the nomenclature for polymer (A), the dashes found in the X, $X'$, and $X''$ divalent groups indicate the side of the group that is attached either to $-CH_2CH_2-R_f-CH_2CH_2-$ in repeating unit (3) or to Z in repeating units (3) and (4).

Polymer (B) can be either polymers comprising 100 mole percent of at least one independently selected repeating unit of the formula

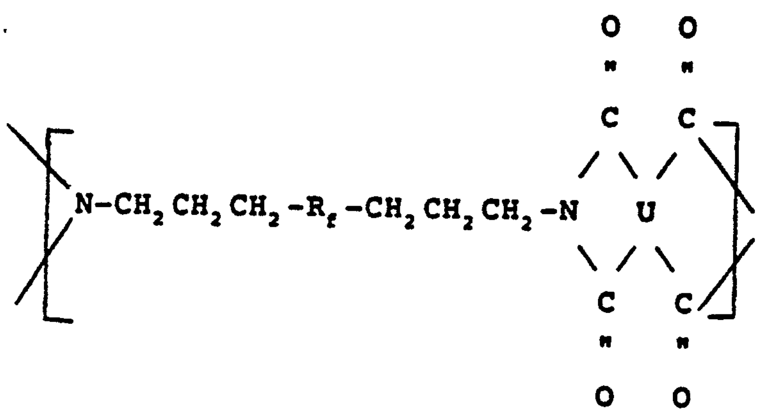

(5)

wherein U is any tetravalent organic radical and $R_f$ is as defined above; or copolymers comprising from 1 to 99 mole percent of at least one independently selected repeating unit of formula (5) and from 99 to 1 mole percent of at least one independently selected repeating unit of the formula

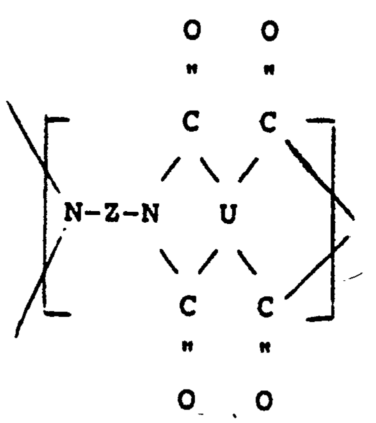

(6)

wherein U and Z are as defined above.

## BRIEF DESCRIPTION OF THE DRAWING

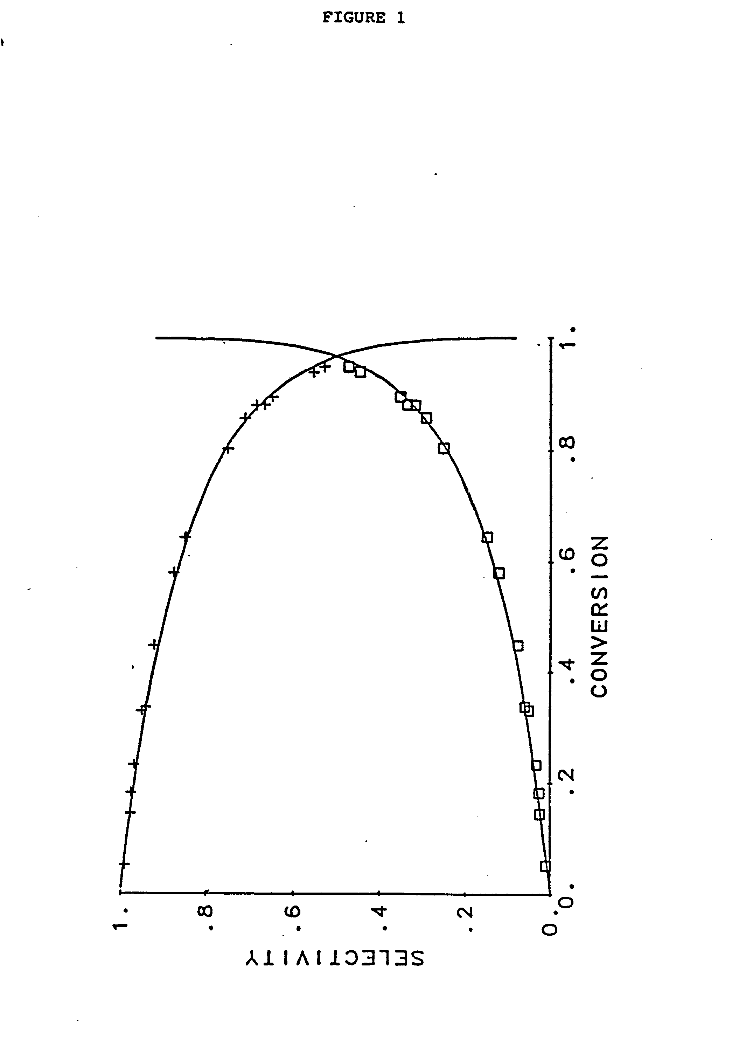

Figure 1 plots the conversion of $CH_2=CH\text{-}CF_2CF_2\text{-}CH=CH_2$ against the selectivity to mononitrile and dinitrile. This shows that the hydrocyanation of a diene is sequential. In the figure, the crosses are experimental points for mononitrile whereas the squares are experimental points for dinitrile. The lines are theoretical fits resulting from the use of a sequential kinetic model

$$\text{diene} \xrightarrow{k_1} \text{mononitrile}$$

$$\text{mononitrile} \xrightarrow{k_2} \text{dinitrile}$$

in the kinetic modeling computer program, GIT (available through the Quantum Chemistry Program Exchange, Department of Chemistry, Indiana University, Bloomington, Indiana). The successful fit illustrates that $k_1$ is 3.5 times greater than $k_2$ and that essentially all dinitrile is derived from mononitrile.

## DETAILED DESCRIPTION OF THE INVENTION

The present invention provides partially fluorinated compounds that are useful as chemical intermediates. The unsaturated nitrile compounds are useful for the preparation of unsaturated acids, amines, and esters. The unsaturated nitriles and esters are intermediates for the production of dinitriles and esternitriles of the present invention. The dinitrile compounds are useful as intermediates for the production of diacids, diesters, diisocyanates, and diamines. The esternitrile compounds can be also converted to esteramines. These difunctional species are useful for the synthesis of partially fluorinated condensation polymers. Polymers prepared with compounds of the invention demonstrate improved hydrolytic stability and altered dielectric constants.

Compounds of the invention include compounds of the formula

$H_2C=CH\text{-}R_f\text{-}CH_2CH_2\text{-}Y$ (1)

wherein Y is selected from the group consisting of -CN, $-CH_2NH_2$, -COOH, $-CH_2OH$, $-CH_2N=C=O$, $-CONH_2$, -CHO, -COCl, and -COOR, wherein R is $C_nH_{2n+1}$ and n is an integer from 1 to 10, inclusive; and $R_f$ is a perfluorinated divalent organic radical selected from the group consisting of linear, branched, carbocyclic, and mixtures thereof having from 1 to about 40 carbon atoms and containing zero to about 20 ether linkages. If ether linkages are present, there will be up to half as many ether linkages as there are total carbon atoms in the $R_f$ chain.

Preferably, Y is -CN. In preferred embodiments, $R_f$ is either the branched and carbocyclic 4,5-(2,2-trifluoromethyl)-1,3-dioxolanediyl

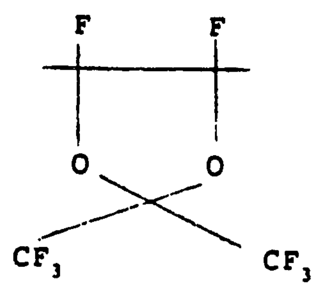

(as shown in Example 14) or of the formula $-(CF_2CF_2)_n-$ wherein n is an integer from 1 to 6.

The invention additionally provides compounds of the formula

$Y'-CH_2CH_2-R_f-CH_2CH_2-Y'$ (2)

wherein Y′ is independently selected from the group consisting of -CN, $-CH_2NH_2$, $-CH_2OH$, $-CH_2N=C=O$, $-CONH_2$, -CHO, -COCl, and -COOR, wherein R is $C_nH_{2n+1}$ and n is an integer from 1 to 10, inclusive; and $R_f$ is as generally defined above.

Preferably, Y′ is -CN. In preferred embodiments, $R_f$ is either the branched and carbocyclic 4,5-(2,2-trifluoromethyl)-1,3-dioxolanediyl or of the formula $-(CF_2CF_2)_n-$ wherein n is an integer from 1 to 6.

Compound (2) can be employed in a condensation type of polymerization reaction to prepare polymers (A) and (B) of the invention. Polymer (A) results when Y′ in compound (2) is $-CH_2NH_2$, $-CH_2OH$, $-CH_2N=C=O$, $-CONH_2$, -COCl, or -COOR, wherein R is $C_nH_{2n+1}$ and n is an integer from 1 to 10. Additionally, $HOOC-CH_2CH_2-R_f-CH_2CH_2-COOH$ can be used to make polymer (A). Polymer (A) can be either polymers comprising 100 mole percent of at least one independently selected repeating unit of the formula

$\{X-CH_2CH_2-R_f-CH_2CH_2-X-X'-Z-X'\}$ (3)

wherein X is independently selected from the group consisting of $NHCH_2-$, $OCH_2-$, -C(=O), and $-CH_2NH-(C=O)$; X′ is independently selected from the group consisting of NH-, O-, and -C(=O), provided that when X is $NHCH_2-$ or $OCH_2-$, X′ must be -C(=O), and also provided that when X is -C(=O) or $-CH_2NH(C=O)$, X′ must be NH- or O-; Z is any divalent organic radical; and $R_f$ is as generally defined above; or copolymers comprising from 1 to 99 mole percent of at least one independently selected repeating unit of formula (3) and from 99 to 1 mole percent of at least one independently selected repeating unit of the formula

$\{X''-Z-X''-X'-Z-X'\}$ (4)

wherein X′ and Z are as defined above, and Z is independently selected, and X″ is independently selected from the same group as X′, provided that when X′ is -C(=O), X″ must be NH- or O-, and also provided that when X′ is NH- or O-, X″ must be -C(=O). In the nomenclature for polymer (A), the dashes found in the X, X′ and X″ divalent groups indicate the side of the group that is attached either to $-CH_2CH_2-R_f-CH_2CH_2-$ in repeating unit (3) or to Z in repeating units (3) and (4).

In repeating unit (3), the preferred $R_f$ is either the branched and carbocyclic 4,5-(2,2-trifluoromethyl)-1,3-dioxolanediyl or of the formula $-(CF_2CF_2)_n-$ wherein n is an integer from 1 to 6. A preferred Z group in repeating units (3) and (4) is a divalent organic radical independently selected from the group consisting of linear, branched, carbocyclic, aromatic, and mixtures thereof having from 1 to about 20 carbon atoms and containing from zero to about 10 ether linkages. If ether linkages are present, there will be up to half as many ether linkages as there are total carbon atoms in the Z chain. Other preferred Z groups are $-CH_2CH_2-R_f-CH_2CH_2-$, provided that the X′ or X″ attached to $-CH_2CH_2-R_f-CH_2CH_2-$ is -C(=O), and $-CH_2CH_2CH_2-R_f-CH_2CH_2CH_2-$, provided that X′ or X″ attached to $-CH_2CH_2CH_2-R_f-CH_2CH_2CH_2-$ is independently selected from the group consisting of -NH and -O, wherein $R_f$ is as generally and specifically defined above. Another preferred Z group is mesogenic divalent organic radicals. Mesogenic divalent organic radicals can include, but are not limited to, divalent radicals of the formula

-V-W-V-

wherein V is a divalent radical independently selected from the group consisting of 1,4-phenylene, 4,4′-biphenylene, naphthylidene, 1,4-[2.2.2]-bicyclooctylidene, and 1,4-cyclohexylidene, and W is a divalent radical selected from the group consisting of -CH=N-, -N(O)=N-, -N=N-, -C(=O)-O-, -C≡C-, -CH=CH-, $-CH=C(CH_3)-$, -C(=O)-NH-, -CH=CH-CH=N-, -CH=CH-C(=O)-O-, and -CH=N-N=CH-. The most preferred embodiment of Z is when Z is selected from the group consisting of $-C_6H_4-$, $-C_{10}H_6-$, and $-(CH_2)_n-$, wherein n is an integer from 2 to 20.

When Y′ is $-CH_2NH_2$ in compound (2), a condensation type of polymerization reaction can yield polymer (B). Polymer (B) can be either polymers comprising 100 mole percent of at least one independently selected repeating unit of the formula

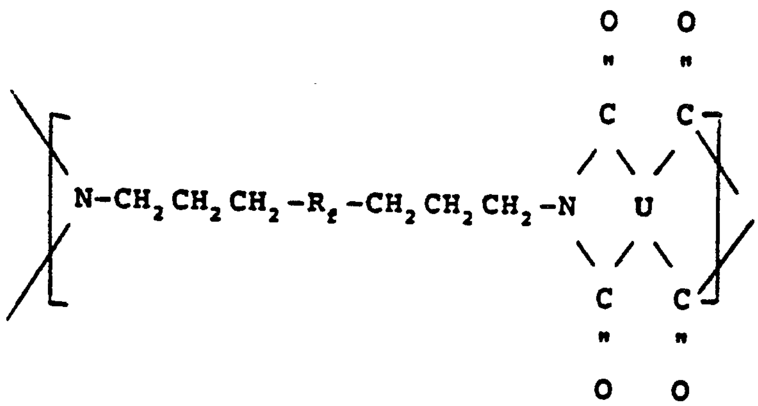

(5)

wherein U is any tetravalent organic radical and $R_f$ is as generally defined above; or copolymers comprising 1 to 99 mole percent of at least one independently selected repeating unit of formula (5) and 99 to 1 mole percent of at least one independently selected repeating unit of the formula

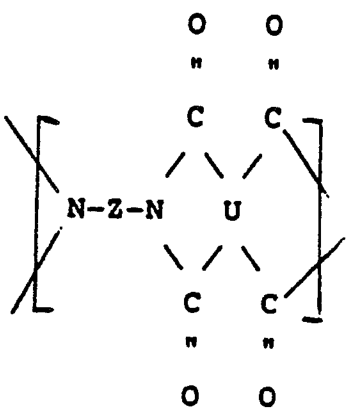

(6)

wherein U and Z are as defined above.

In repeating unit (5), the preferred $R_f$ is either the branched and carbocyclic 4,5-(2,2-trifluoromethyl)-1,3-dioxolanediyl or of the formula $-(CF_2CF_2)_n-$ wherein n is an integer from 1 to 6. A preferred U group in repeating units (5) and (6) is a divalent organic radical selected from the group consisting of linear, branched, carbocyclic, aromatic, and mixtures thereof having from about 6 to about 20 carbon atoms and containing from 0 to about 5 linkages selected from the group consisting of ether and sulfonyl. Most preferred embodiments of U are 1,2,4,5-benzenetetrayl, 2,2-bis(3,4-phenylene)-propane and 2,2-bis(3,4-phenylene)-1,1,1,3,3,3-hexafluoropropane. In repeating unit (6), a preferred Z group is a divalent organic radical independently selected from the group consisting of linear, branched, carbocyclic, aromatic, and mixtures thereof having from 1 to about 20 carbon atoms and containing from zero to about 10 ether linkages. If ether linkages are present, there will be up to half as many ether linkages as there are total carbon atoms in the Z chain. Other preferred embodiments of Z are 4,4′-diphenylene ether, m-phenylene, and p-phenylene.

Compounds (1) of the invention wherein Y is -CN can be made by contacting $CH_2=CH(R_f)_nCH=CH_2$ with HCN in the presence of a nickel catalyst and a Lewis acid promoter such as zinc chloride by a process similar to that described in U.S. 3,496,217, the disclosure of which is incorporated herein by reference. The diene starting compound can be prepared by the method disclosed in Kim, et al., J. Fluor. Chem. 1:203 (1971) or by other conventional chemistry.

Compound (2) of the invention wherein one or both values for Y′ is -CN can be made by contacting compound (1) with HCN in a manner similar to that described above. The addition of two equivalents of HCN to the diene to make compound (2), without isolation of the intermediate compound (1), is sequential, i.e., the dinitrile is produced only from the mononitrile. The kinetic analysis shown in Figure 1 wherein $R_f$ is $-CF_2CF_2-$ illustrates this and shows that compound (2) is produced only from compound (1). Similar analyses have been carried out for many other $R_f$ values and produced the same result.

Other Y values for compounds (1) and (2) result from converting the -CN group to other useful functional groups by conventional chemistry. For example, the -CN group can be converted to the $-CH_2NH_2$

group by reaction with hydrogen in the presence of ammonia and Raney cobalt. The $-CONH_2$ group can be prepared by reaction of the -CN group with water in the presence of sulfuric acid. The -COOH group can be prepared by heating the -CN group in 50% sulfuric acid in water. The -COCl group can be made by reacting the -COOH group with a reagent such as thionyl chloride or phosporus pentachloride. The -COOR group can be made by heating the -CN group in ROH solution in the presence of sulfuric acid. The -CHO group can be made by partially reducing the -COOR group with a reagent such as lithium tri-t-butoxyaluminum hydride. The $-CH_2OH$ group can be made by the reduction of the -COOR, or -COOH group, e.g., by reacting with lithium aluminum hydride. The $-CH_2N=C=O$ group can be prepared by the reaction of the $-CH_2NH_2$ group with phosgene.

Compound (1) of the invention has utility for the preparation of the difunctional compound (2). Compound (2) of the invention is useful as an intermediate for the synthesis of polymer (A) and polymer (B) of the invention by condensation type polymerization. Partially fluorinated polyamides can be made by heating a salt of the desired acid and amine and allowing the water formed in the reaction to distill off. Higher molecular weights can be obtained by vacuum finishing the molten polymer using standard techniques. Polyesters of the invention can be made by heating a partially fluorinated free acid with the diacetate of a diol using calcium acetate as a catalyst. Polyimides of the invention can be made by adding a dianhydride to a solution of partially fluorinated diamine in a solvent such as dimethylacetamide (DMAC). The resulting amid-acid can be cast as a film and imidized by heating in a vacuum.

Methods for conducting condensation type polymerization are well known in the art. For example, 4,4,5,5-tetrafluorosuberic acid can be polymerized with hexamethylenediamine by heating a salt prepared from these materials. Also, 4,4,5,5,6,6,7,7-octofluorosebecic acid can be polymerized with 1,4-butanediol diacetate by heating in the presence of calcium acetate as a catalyst. Most examples of polymer (A) are moldable or melt processible, and most examples of polymer (B) can be processed like other polyimides.

It has been found that incorporation of compound (2) as a monomer improves the thermal characteristics of the resulting polymers (A) and (B). For example, poly(hexamethylenesuberamide) has a melting point of 232° C, whereas poly(hexamethylene-4,4,5,5-tetrafluorosuberamide) has a melting point of 268° C. This increase in melting point is evident also in polyesters. For example, poly(tetramethylenesuberate) melts at 60° C, whereas poly(tetramethylene-4,4,5,5-tetrafluorosuberate) melts at 123° C.

The partially fluorinated compounds of the invention can increase the anisotropic range of polymers in which they are incorporated as a monomer. For example, poly(oxydecanedioyloxy-1,4-phenylene-2-methylvinylene-1,4-phenylene) melts at 210° C to a nematic anisotropic phase. The resulting melt becomes isotropic at 254° C, with an anistropic range of 44° C. In contrast, poly(oxyoctafluorodecanedioyloxy-1,4-phenylene-2-methylvinylene-1,4-phenylene) melts at 153° C to a more highly ordered, smectic anisotopic state. The resulting melt becomes isotropic at 280° C, with an anisotropic range of 127° C.

The partially fluorinated compounds of this invention can alter the solubility properties of polymers in which they are incorporated as a monomer. For example, where poly(oxydecanedioyloxy-1,4-phenylene-2-methylvinylene-1,4-phenylene) is soluble in chloroform, poly(oxyoctafluorodecanedioyloxy-1,4-phenylene-2-methylvinylene-1,4-phenylene) is insoluble in all common organic non-acid solvents. The compounds and polymers of the present invention are described in the following examples.

## EXAMPLES

### General Procedure

In these Examples, compounds (1) and (2) were prepared according to the following general procedure. A 3-necked, round-bottom flask equipped with an overhead mechanical stirrer, reflux condenser (used with a low temperature circulation bath), nitrogen bubbler, and rubber septum was charged under nitrogen with the specified catalyst components, toluene, and the desired diene. The resulting mixture was heated in a 50° C oil bath. Hydrogen cyanide was fed to the mixture by vapor transfer by passing nitrogen gas through liquid hydrogen cyanide cooled to 0° C in an ice bath. Under these conditions, the saturated vapor mixture was approximately 30-40% hydrogen cyanide. The hydrogen cyanide vapor was admitted to the reaction vessel via a syringe needle passed through the rubber septum and placed slightly above the liquid level. The vapor absorbed into the reaction mixture. The reflux condenser was cooled to -14° C to prevent escape of nonabsorbed hydrogen cyanide. Progress of reactions was monitored by removing samples with a

syringe, diluting with acetone, and analyzing by capillary gas chromatography (cross-linked methyl silicone column, 25 m, 0.2 mm inner diameter).

## Example 1

### Preparation of $H_2C=CH-(CF_2)_2-CH_2CH_2-CN$ and $NC-CH_2CH_2-(CF_2)_2-CH_2CH_2-CN$

A reaction mixture comprised of Ni(p-TTP)$_4$ (0.30 g; 0.20 mmol), p-TTP (0.25 mL; 0.82 mmol) (wherein p-TTP is tri-p-tolylphosphite), toluene (8.0 mL), $BPh_3$ (0.05 g; 0.2 mmol), and $CH_2=CH(CF_2)_2CH=CH_2$ - (2.24 g; 0.45 mmol) was treated at 50°C with hydrogen cyanide (nitrogen flow = 4 mL/min) as described in the general procedure above. Two new peaks that grew with time were observed by capillary gas chromatography (CGC). White solid began to form in the reaction mixture after 1.5 hours. The reaction was stopped, and the white solid was found to be associated with the longer retention time new peak. Subsequent analysis revealed that the new Peaks were associated with $H_2C=CH-(CF_2)_2-CH_2CH_2-CN$ and $NC-CH_2CH_2-(CF_2)_2-CH_2CH_2-CN$ (the white solid).

## Example 2

### Preparation of $H_2C=CH-(CF_2)_2-CH_2CH_2-CN$ and $NC-CH_2CH_2-(CF_2)_2-CH_2CH_2-CN$

A reaction mixture comprised of Ni(TTP)$_4$ (7.0 mL, 5.6 mmol) (wherein TTP is tri-m,p- tolylphosphite), p-TTP (5.0 mL; 16.3 mmol), toluene (125 mL), $ZnCl_2$ (0.50 g; 3.7 mmol), and a mixture of $CH_2=CH(CF_2)_2CH=CH_2$ and acetonitrile (55 g; 80:20) was treated at 50°C with hydrogen cyanide (nitrogen flow = 17 mL/min for 7.5 hours and then 7 mL/min until complete). The reaction mixture was cooled, resulting in precipitation of a white solid. The mixture was filtered to remove the crude dinitrile. The solvent was then removed from the filtrate in vacuo causing more dinitrile to precipitate. The dinitrile was filtered and washed with diethylether. The precipitates were combined and recrystallized from methanol, yielding 25.4 g (34%) of white crystals, m.p. 109-110°C. Fluorine NMR -116.7, m. Proton NMR 2-2.7, c. IR 2950 $cm^{-1}$, w, (C-H); 2260 $cm^{-1}$, m, (C≡N); 1180 $cm^{-1}$, s, (C-F). The ether was removed from the filtrate in vacuo and the resulting oil distilled. $H_2C=CHC-(CF_2)_2-CH_2CH_2-CN$ was collected, b.p. 60°C at 5mm Hg, 37 g (57%). Proton NMR 6.7-7.2, c, (olefin); 5.6-6.2, c, (olefin); 2.1-2.8, c, (alkyl). Fluorine NMR -115.4, c, 2F; -116.75, c, 2F. IR (neat) 3005 $cm^{-1}$, w, (C-H olefin); 2970 $cm^{-1}$, w, (C-H alkyl); 2226 $cm^{-1}$, m, (C≡N); 1655, m, (C=C); 1110 $cm^{-1}$, vs, (C-F).

| Elemental Analysis for $NC-CH_2CH_2-(CF_2)_2-CH_2CH_2-CN$: | |
|---|---|
| Calculated: | %C 46.16; %H 3.87; %F 36.51 |
| Found: | %C 45.90; %H 4.05; %F 36.22 |

## Example 3

### Preparation of $NC-CH_2CH_2-(CF_2)_4-CH_2CH_2-CN$

A reaction mixture comprised of Ni(TTP)$_4$ (15.0 mL, 12.2 mmol), p-TTP (10 mL; 32.6 mmol), toluene (250 mL), acetonitrile (20 mL), $ZnCl_2$ (1.0 g; 7.4 mmol), and $CH_2=CH(CF_2)_4CH=CH_2$ (161.5 g; 636 mmol) was treated at 50°C with hydrogen cyanide (nitrogen flow = 40 mL/min for 1 hour, 32 mL/min for 25 hours,

and 16 mL/min for 3 hours). The reaction mixture was cooled to -30 °C for about 18 hours and crude NC-$CH_2CH_2$-$(CF_2)_4$-$CH_2CH_2$-CN was isolated by filtration. The solvent was removed from the resulting filtrate in vacuo resulting in more white solid. The solids were redissolved in acetone and precipitated by addition of pentane. The solid was isolated by filtration and recrystallized from methanol producing white crystals (168 g; 86% yield) m.p. 66-68 °C. Fluorine NMR -116.0, c, 2F; -124.0, dd, 2F.

| Elemental Analysis for NC-$CH_2CH_2$-$(CF_2)_4$-$CH_2CH_2$-CN: | |
|---|---|
| Calculated: | %C 38.97; %H 2.62; %F 49.32 |
| Found: | %C 38.73; %H 2.75; %F 49.05 |

## Example 4

### Preparation of NC-$CH_2$ $CH_2$-$(CF_2)_6$-$CH_2CH_2$-CN

A reaction mixture comprised of Ni(p-TTP)$_4$ (1.0 g; 0.68 mmol), p-TTP (0.75 mL; 2.4 mmol), toluene (15 mL), $BPh_3$ (0.10 g; 0.41 mmol), and $CH_2=CH(CF_2)_6CH=CH_2$ (9.0 mL; 13.3 g; 37.5 mmol) was treated at 50 °C with hydrogen cyanide (nitrogen flow = 4 mL/min for 4 hours, 3.5 mL/min for 4 hours, and 2 mL/min for about 18 hours). The entire reaction mixture was redissolved in acetone and filtered to remove the nickel containing compounds. Hexanes were added to precipitate the product. The product was filtered and the solids recrystallized from methanol to give white crystals of NC-$CH_2CH_2$-$(CF_2)_6$-$CH_2CH_2$-CN (6.8 g) m.p. 80-81 °C. Proton NMR 2-2.8, c. Fluorine NMR -115.8, m, 2F;-122.3, m, 2F; -124.0, m, 2F.

| Elemental Analysis for NC-$CH_2CH_2$-$(CF_2)_6$-$CH_2CH_2$-CN: | |
|---|---|
| Calculated: | %C 33.09; %H 1.59; %F 59.81 |
| Found: | %C 32.91; %H 1.58; %F 59.46 |

## Example 5

### Preparation of NC-$CH_2CH_2$-$(CF_2)_8$-$CH_2CH_2$-CN

A reaction mixture comprised of Ni(TTP)$_4$ (7.0 mL, 5.6 mmol), p-TTP (5.0 mL; 16.3 mmol), toluene (130 mL), acetonitrile (10 mL), $ZnCl_2$ (0.50 g; 3.7 mmol), and $CH_2=CH(CF_2)_8CH=CH_2$ (50 g, 110 mmol) was treated at 50 °C with hydrogen cyanide (nitrogen flow = 18 mL/min for 3 hours and 13 mL/min for 5 hours). The mixture was diluted with hexanes and cooled to -30 °C to crystallize the NC-$CH_2CH_2$-$(CF_2)_8$-$CH_2CH_2$-CN. The crude product was dissolved in acetone and filtered through Celite to remove the spent catalyst. The acetone was then removed, and the product was recrystallized from methanol. The yield was 46 g (82%).

## Example 6

Preparation of $NC-CH_2CH_2-(CF_2)_8-CH_2CH_2-CN$

A reaction mixture comprised of $Ni(TTP)_4$ (20 mL, 16.3 mmol), p-TTP (10 mL; 32.6 mmol), toluene (300 mL), acetonitrile (10 mL), $ZnCl_2$ (1.0 g; 7.4 mmol), and $CH_2=CH(CF_2)_8CH=CH_2$ (200 g; 440 mmol) was treated at 50 °C with hydrogen cyanide (nitrogen flow = 35 mL/min) for 26 hours. The mixture was diluted with hexanes and cooled to -30 °C to crystallize $NC-CH_2CH_2-(CF_2)_8-CH_2CH_2-CN$. The crude product was dissolved in acetone and filtered through Celite to remove the spent catalyst. The acetone was then removed, and the product was recrystallized from methanol. The yield was 202 g (90%).

## Example 7

Preparation of $HOOC-CH_2CH_2-(CF_2)_2-CH_2CH_2-COOH$

$NC-CH_2CH_2-(CF_2)_2-CH_2CH_2-CN$ (1.0 g) was added to a solution of 50 mL water and 50 mL of concentrated sulfuric acid. The resulting mixture was heated to 130 °C for 18 hours. Upon cooling to 0 °C, white crystals formed. The crystals were filtered, washed with cold water, and recrystallized from water. Yield 0.8 g white crystals, m.p. 204-206 °C. Proton NMR (acetone) 2.6, c. Fluorine NMR -115.8, dt. IR (KBr) 3500-2500, $cm^{-1}$ br, (O-H); 1715 $cm^{-1}$, s, (C=O); 1200 $cm^{-1}$, vs, (C-F).

## Example 8

Preparation of $HOOC-CH_2CH_2-(CF_2)_4-CH_2CH_2-COOH$

$NC-CH_2CH_2-(CF_2)_4-CH_2CH_2-CN$ (100 g) was added to 500 mL of water. Concentrated sulfuric acid (700 mL) was slowly added, and the resulting reaction mixture was heated to 150 °C for about 18 hours. Upon cooling to 0 °C, crystals formed. The crystals were filtered, washed with water, and recrystallized from water with a hot filtration through Celite. Yield 55 g of white crystals. IR 3500-2400 $cm^{-1}$, br; 1710 $cm^{-1}$, s; 1250-1150 $cm^{-1}$, s.

## Example 9

Preparation of $H_3COOC-CH_2CH_2-(CF_2)_2-CH_2CH_2-COOCH_3$

$HOOC-CH_2CH_2-(CF_2)_2-CH_2CH_2-COOH$ (25 g) was dissolved in 100 mL methanol. Concentrated sulfuric acid (10 mL) was added, the resulting solution was refluxed for 4 hours, and then stirred at ambient temperature for 2 days. The solution was cooled to 0 °C and crystals formed. The crystals were filtered, washed with cold methanol, and recrystallized from methanol. The resulting filtrate was added to 500 mL of water and extracted with 4 100 mL portions of ether. The ether layers were washed with 100 mL water, 2 100 mL portions of 10% $Na_2CO_3$, 2 100 mL portions of water, and 100 mL of brine. The resulting solution was dried over $MgSO_4$ and then stripped to give white crystals that were recrystallized from methanol. Yield 23 g white crystals, m.p. 64-66 °C. IR (Ksr) 1740 $cm^{-1}$, s, (C=O).

## Example 10

Preparation of $H_3COOC-CH_2CH_2-(CF_2)_2-CH_2CH_2-COOCH_3$

$NC-CH_2CH_2-(CF_2)_2-CH_2CH_2-CN$ (50 g) was dissolved in 200 mL of methanol. Concentrated sulfuric acid (150 mL) was added slowly and the resulting solution was refluxed for 6 hours. The solution was then dumped into 1 L of ice. The resulting mixture was extracted with 3 200 mL portions of ether. The ether layers were washed with 2 100 mL portions of water and 50 mL of brine. The solution was dryed over $MgSO_4$ and then stripped to give white crystals that were recrystallized from methanol. Yield 57 g (87%), m.p. 64-65 °C. IR (KBr) 2980 $cm^{-1}$, m; 1740 $cm^{-1}$, s; 1200 $cm^{-1}$, s.

## Example 11

### Preparation of $NC-CH_2CH_2-(CF_2)_6-CH_2CH_2-CN$

The method described in Example 6 was substantially repeated except that $CH_2=CH(CF_2)_6CH=CH_2$ - (200 g; 565 mmol) was hydrocyanated and $NC-CH_2CH_2-(CF_2)_6-CH_2CH_2-CN$ was produced. Yield 215 g (93%).

## Example 12

### Preparation of $H_2N-CH_2CH_2CH_2-(CF_2)_2-CH_2CH_2CH_2-NH_2$

$NC-CH_2CH_2-(CF_2)_2-CH_2CH_2-CN$ (4.8 g) was dissolved in 200 mL of THF. This solution was placed in a 400 mL bomb. The bomb was cooled and evacuated. Ammonia (50 g) was added to the bomb and then hydrogen was added until the pressure was 1500 psi at ambient temperature. The bomb was sealed and heated to 110 °C for 10 hours. The bomb was cooled, and the gasses were bled off. The resulting solution was filtered through Celite and then stripped to a pale yellow oil. The oil was dissolved in 300 mL of ether, and HCl gas was bubbled through the resulting solution. A white precipitate formed that was filtered, washed with ether, and dried. The precipitate was recrystallized from a mixture of methanol and ethanol to give 5 g of white crystals. Fluorine NMR ($D_2O$) -114.5, t. Proton NMR 3.0, t, 2H; 2.0, c, 4H.

## Example 13

### Preparation of $H_2N-CH_2CH_2CH_2-(CF_2)_8-CH_2CH_2CH_2-NH_2$

$NC-CH_2CH_2-(CF_2)_8-CH_2CH_2-CN$ (50 g) was placed into a 400 mL bomb, along with 200 mL of THF and approximately 5 g of Raney cobalt. The bomb was sealed and 50 g of ammonia were added. Hydrogen was added to a pressure of 500 psi at ambient temperature. The bomb was heated to 110 °C and the hydrogen pressure was adjusted to 1500 psi. The temperature and hydrogen pressure were held constant for 18 hours. The bomb was cooled, and the gasses were bled off. The solution was filtered through Celite, and the solvent was removed to give 13 g of a white waxy solid. Proton NMR ($CDCl_3$/F11) 2.7, t, 38; 1.5-2.5, c, 87; 1.0, s, 42. Fluorine NMR -114.6, 2F; -122.3, 4F; -124.0, 2F. IR (KBr) 3400 $cm^{-1}$, m; 3300 $cm^{-1}$, m; 3200 $cm^{-1}$, m; 2960 $cm^{-1}$, m; 1200 $cm^{-1}$, vs.

## Example 14

**Preparation of** 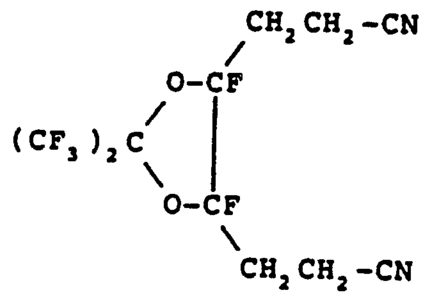

A reaction mixture comprised of $Ni(p\text{-}TTP)_4$ (0.25 g; 0.17 mmol), p-TTP (0.5 mL; 1.6 mmol), toluene (5 mL), acetonitrile (0.2 mL), $ZnCl_2$ (0.005 g; 0.038 mmol), and

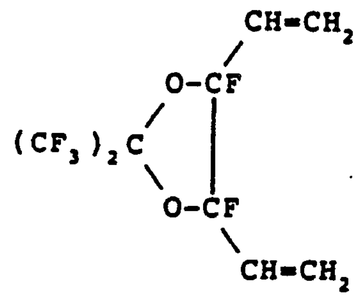

(1.0 g; 3.35 mmol) was treated at 50 °C with hydrogen cyanide (nitrogen flow = 3 mL/min) for 20 hours. The resulting reaction mixture was cooled and filtered through Celite. Gas chromatography analysis showed two new products. High resolution GC/MS showed that the first product was

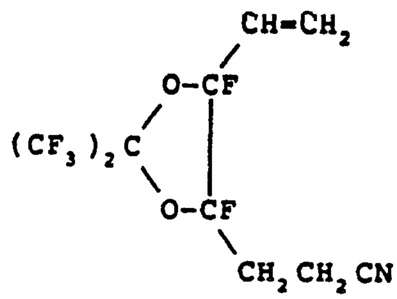

and the second product was

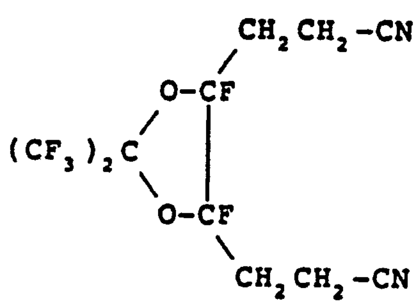

IR (reaction mixture) 2280 $cm^{-1}$, m; 2255 $cm^{-1}$, m; 1300-1100 $cm^{-1}$, vs.

Example 15

Preparation of Partially Fluorinated Polyamide Polymer (A)

A 500 mL round-bottom flask was equipped with an overhead stirrer, a nitrogen inlet, and a Liebig's condenser topped with a still head. The flask was charged with 35 g of crystals of the salt of tetrafluorosuberic acid and hexamethylenediamine prepared according to the following procedure. Tetrafluorosuberic acid (25.00 g) was dissolved in 250 mL of hot ethanol, and 11.79 g of hexamethylenediamine were dissolved in 100 mL of hot ethanol. The resulting solutions were combined to produce a suspension containing white crystals. The suspension was stirred for 10 minutes, cooled to 0° C, filtered, washed with ethanol, and dried in vacuo to give 35.97 g of white crystals. This material was immediately charged to the flask as described above.

The flask was heated to 220° C, and the water formed was allowed to reflux. Over a period of about 1 hour, the temperature of the flask was raised to 290° C. The water in the condenser was drained. The water formed in the reaction was allowed to distill off. Over the next hour, the temperature of the flask was slowly raised to 300° C, giving a viscous amber melt. The melt was allowed to cool, giving 30 g of an amber polymer.

The inherent viscosity of the polymer in a formic acid solution was 1.07 dl/g. The polymer formed strong melt pressed films that had an aqueous contact angle of 74°. The polymer was also spun to form strong fibers. The melting point of the polymer was 268° C, as compared to 232° C for the melting point of the nonfluorinated polymer.

## Example 16

Preparation of Partially Fluorinated Polyester Polymer (A)

A small polymer tube with a side arm was charged with 5.00 g of dimethyltetrafluorosuberate, 1.81 g of butanediol, 3 mg of calcium acetate, and 1.5 mg of antimony oxide. The resuting mixture was degassed and heated to 200° C. A needle with a slow flow of nitrogen was put at the bottom of the melt and the temperature was raised to 275° C over a period of 3 hours and then held there for 16 hours. During this time, first methanol and then butanediol slowly distilled from the resulting melt. The pressure was then reduced to about 1 mm Hg for a period of 30 minutes. The melt was cooled to form an opaque white solid polymer. Fibers and melt pressed films could be made from the polymer. The inherent viscosity of the melt in a chloroform solution was 0.28 dl/g.

The polymer had a melting point of 123° C as compared to 60° C for nonfluorinated polymer.

## EXAMPLE 17

Preparation of Partially Fluorinated Polyimide Polymer (B)

Dodecafluoro-1,12-dodecadiamine (8.975 g) was dissolved in about 100 mL of N-methylpyrrolidone (NMP) in a 500 mL 3-necked flask equipped with an overhead stirrer and a nitrogen inlet. Pyromelliticdianhydride (4.567 g) was added in several batches, producing a viscous solution containing some gel. About 100 mL of NMP were added to dissolve some of the gel. The resulting solution was filtered through a 0.2 micron filter and cast on a glass plate. The solvent was evaporated at 75° C for about 18 hours. The polymer was imidized by heating in vacuo at 150° C for about 16 hours and then at 175° C for 4 hours to give a tough clear film. The polymer had a melting point of 341° C and did not decompose below 450° C.

## Example 18

Preparation of Partially Fluorinated Liquid Crystalline Polyester Polymer (A)

Alpha-methylstilbenediol diacetate (2.62 g), hexadecafluorododecane-1,12-dicarboxylate (5.0 g), and 0.01 g sodium acetate were added to a polymer tube with a side arm. The resulting mixture was melted in a metal bath at 200° C, and a needle with a slow purge of nitrogen was placed at the bottom of the resulting melt. The melt was held at 200° C for 18 hours while acetic acid distilled off. The temperature was then raised to 250° C for 5 hours. The nitrogen purge was stopped, and the pressure was reduced to about 1 mm Hg for 1 hour. The resulting combination was cooled to give an off-white solid polymer. The polymer melted at 230° C to a smectic anisotropic state. The resulting melt remained anisotropic until a temperature of 310° C was reached, at which point the melt was isotropic. Upon cooling, the smectic state returned at about 270° C and remained anisotropic until the polymer hardened at about 220° C.

This polymer had an enantiotropic anisotropic range of 80° C, as opposed to a monotropic anisotropic range of 7° C for the corresponding nonfluorinated polymer. The anisotropic state for the fluorinated polymer was in a more highly ordered smectic state, as compared to the nematic anisotropic state of the nonfluorinated polymer.

Example 19

Preparation of Partially Fluorinated Liquid Crystalline Polyester Copolymer (A)

4,4′-diacetoxybenzoyl phenol (2.00 g), tetrafluorosuberic acid (1.91 g), and 0.01 g calcium acetate were placed in a small polymer tube with a side arm. The resulting mixture was heated to 250° C and nitrogen was slowly purged through the resulting melt for about 18 hours. The melt was cooled to give an off-white polymer. Fibers could be pulled from the melt. The polymer melted at 215° C to an anisotropic phase. It remained anisotropic upon heating to 400° C. This yielded an anisotropic range of greater than 185° C, as compared to the nonfluorinated polymer, which had an anisotropic range of 115° C.

**Claims**

1. A compound of the formula

$H_2C=CH-R_f-CH_2CH_2-Y$ (1)

wherein Y is independently selected from the group consisting of -CN, $-CH_2NH_2$, -COOH, $-CH_2OH$, $-CH_2N=C=O$, $-CONH_2$, -CHO, -COCl, and -COOR, wherein R is $C_nH_{2n+1}$ and n is an integer from 1 to 10, inclusive; and $R_f$ is a perfluorinated divalent organic radical selected from the group consisting of linear, branched, carbocyclic, and mixtures thereof having from 1 to about 40 carbon atoms and containing from zero to about 20 ether linkages.

2. A compound according to Claim 1 wherein Y is -CN.

3. A compound according to Claims 1 or 2 wherein $R_f$ is the branched and carbocyclic 4,5-(2,2-trifluoromethyl)-1,3-dioxolanediyl.

4. A compound according to Claims 1 or 2 wherein $R_f$ is $-(CF_2CF_2)_n-$ and n is an integer from 1 to 6.

5. A compound of the formula

$Y'-CH_2CH_2-R_f-CH_2CH_2-Y'$ (2)

wherein Y′ is independently selected from the group consisting of -CN, $-CH_2NH_2$, $-CH_2OH$, $-CH_2N=C=O$, $-CONH_2$, -CHO, -COCl, and -COOR, wherein R is $C_nH_{2n+1}$ and n is an integer from 1 to 10, inclusive; and $R_f$ is a perfluorinated divalent organic radical selected from the group consisting of linear, branched, carbocyclic, and mixtures thereof having from 1 to about 40 carbon atoms and containing from zero to about 20 ether linkages.

6. A compound according to Claim 5 wherein Y′ is -CN.

7. A compound according to Claims 5 or 6 wherein $R_f$ is the branched and carbocyclic 4,5-(2,2-trifluoromethyl)-1,3-dioxolanediyl.

8. A compound according to Claims 5 or 6 wherein $R_f$ is $-(CF_2CF_2)_n-$ and n is an integer from 1 to 6.

9. A polymer (A) comprising 100 mole percent of at least one independently selected repeating unit of the formula

$\{X-CH_2CH_2-R_f-CH_2CH_2-X-X'-Z-X'\}$ (3)

wherein X is independently selected from the group consisting of $NHCH_2-$, $OCH_2-$, -C(=O), and $-CH_2NH-(C=O)$; X′ is independently selected from the group consisting of NH-, O-, and -C(=O), provided that when X is $NHCH_2-$ or $OCH_2-$, X′ must be -C(=O), and also provided that when X is -C(=O) or $-CH_2NH(C=O)$, X′

must be NH- or O-; Z is any divalent organic radical; and $R_f$ is a perfluorinated divalent organic radical selected from the group consisting of linear, branched, carbocyclic, and mixtures thereof having from 1 to about 40 carbon atoms and containing from zero to about 20 ether linkages.

10. A polymer (A) according to Claim 9 wherein $R_f$ is the branched and carbocyclic 4,5-(2,2-trifluoromethyl)-1,3-dioxolanediyl.

11. A polymer (A) according to Claim 9 wherein $R_f$ is $-(CF_2CF_2)_n-$ and n is an integer from 1 to 6.

12. A polymer (A) according to Claims 9, 10, or 11 wherein Z is selected from the group consisting of linear, branched, carbocyclic, aromatic, and mixtures thereof having from 1 to about 20 carbon atoms and containing from zero to about 10 ether linkages.

13. A polymer (A) according to Claim 12 wherein Z is selected from the group consisting of $-C_6H_4-$, $-C_{10}H_6-$, and $-(CH_2)_n-$ and n is an integer from 2 to 20.

14. A polymer (A) according to Claims 9, 10, or 11 wherein Z is $-CH_2CH_2-R_f'-CH_2CH_2-$; $X'$ is -C(=O); and $R_f'$ is a perfluorinated divalent organic radical selected from the group consisting of linear, branched, carbocyclic, and mixtures thereof having from 1 to about 40 carbon atoms and containing from zero to about 20 ether linkages.

15. A polymer (A) according to Claim 14 wherein $R_f'$ is the branched and carbocyclic 4,5-(2,2-trifluoromethyl)-1,3-dioxolanediyl.

16. A polymer (A) according to Claim 14 wherein $R_f'$ is $-(CF_2CF_2)_n-$ and n is an integer from 1 to 6.

17. A polymer (A) according to Claims 9, 10, or 11 wherein Z is $-CH_2CH_2CH_2-R_f'-CH_2CH_2CH_2-$; $X'$ is independently selected from the group consisting of -NH and -O; and $R_f'$ is a perfluorinated divalent organic radical selected from the group consisting of linear, branched, carbocyclic, and mixtures thereof having from 1 to about 40 carbon atoms and containing from zero to about 20 ether linkages.

18. A polymer (A) according to Claim 17 wherein $R_f'$, is the branched and carbocyclic 4,5-(2,2-trifluoromethyl)-1,3-dioxolanediyl.

19. A polymer (A) according to Claim 17 wherein $R_f'$ is $-(CF_2CF_2)_n-$ and n is an integer from 1 to 6.

20. A polymer (A) according to Claims 9, 10, or 11 wherein Z is any mesogenic divalent organic radical.

21. A polymer (A) according to Claim 20 wherein Z is of the formula

-V-W-V-

wherein V is a divalent radical independently selected from the group consisting of 1,4-phenylene, 4,4'-biphenylene, naphthylidene, 1,4-[2.2.2]-bicyclooctylidene, and 1,4-cyclohexylidene; and W is a divalent radical selected from the group consisting of -CH=N-, -N(O)=N-, -N=N-, -C(=O)-O-, -C=C-, -CH=CH-, $-CH=C(CH_3)-$, -C(=O)-NH-, -CH=CH-CH=N-, -CH=CH-C(=O)-O-, and -CH=N-N=CH-.

22. A copolymer (A) comprising from 1 to 99 mole percent of at least one independently selected repeating unit of the formula

$\{X-CH_2CH_2-R_f-CH_2CH_2-X-X'-Z-X'\}$ 3)

wherein X is independently selected from the group consisting of $NHCH_2-$, $OCH_2-$, -C(=O), and $-CH_2NH-(C=O)$; $X'$ is independently selected from the group consisting of NH-, O-, and -C(=O), provided that when X is $NHCH_2-$ or $OCH_2-$, $X'$ must be -C(=O), and also provided that when X is -C(=O) or $-CH_2NH(C=O)$, $X'$ must be NH- or O-; Z is any divalent organic radical; and $R_f$ is a perfluorinated divalent organic radical selected from the group consisting of linear, branched, carbocyclic, and mixtures thereof having from 1 to about 40 carbon atoms and containing from zero to about 20 ether linkages; and from 99 to 1 mole percent of at least one independently selected repeating unit of the formula

$\{X''-Z'-X''-X'-Z'-X'\}$ (4)

wherein $X'$ is independently selected from the group consisting of NH-, O-, and -C(=O); $X''$ is independently selected from the group consisting of NH-, O-, and -C(=O), provided that when $X'$ is -C(=O), $X''$ must be NH- or O-, and also provided that when $X'$ is NH- or O-, $X''$ must be -C(=O); and $Z'$ is any divalent organic radical independently selected.

23. A copolymer (A) according to Claim 22 wherein $R_f$ is the branched and carbocyclic 4,5-(2,2-trifluoromethyl)-1,3-dioxolanediyl.

24. A copolymer (A) according to Claim 22 wherein $R_f$ is $-(CF_2CF_2)_n-$ and n is an integer from 1 to 6.

25. A copolymer (A) according to Claims 22, 23, or 24 wherein Z in repeating unit (3) and $Z'$ in repeating unit (4) are independently selected from the group consisting of linear, branched, carbocyclic, aromatic, and mixtures thereof having from 1 to about 20 carbon atoms and containing from zero to about 10 ether linkages.

26. A copolymer (A) according to Claim 25 wherein at least one of Z or $Z'$ is selected from the group consisting of $-C_6H_4-$, $-C_{10}H_6-$, and $-(CH_2)_n-$ and n is an integer from 2 to 20.

27. A copolymer (A) according to Claims 22, 23, or 24 wherein at least one of Z or $Z'$ is $-CH_2CH_2-R_f'-CH_2CH_2-$; $X'$ is -C(=O); and $R_f'$ is a perfluorinated divalent organic radical selected from the group

consisting of linear, branched, carbocyclic, and mixtures thereof having from 1 to about 40 carbon atoms and containing from zero to about 20 ether linkages.

28. A copolymer (A) according to Claim 27 wherein $R_f'$ is the branched and carbocyclic 4,5-(2,2-trifluoromethyl)-1,3-dioxolanediyl.

29. A copolymer (A) according to Claim 27 wherein $R_f'$ is $-(CF_2CF_2)_n-$ and n is an integer from 1 to 6.

30. A copolymer (A) according to Claims 22, 23, or 24 wherein at least one of Z or Z′ is $-CH_2CH_2CH_2-R_f'-CH_2CH_2CH_2-$; X′ is independently selected from the group consisting of -NH and -O; and $R_f'$ is a perfluorinated divalent organic radical selected from the group consisting of linear, branched, carbocyclic, and mixtures thereof having from 1 to about 40 carbon atoms and containing from zero to about 20 ether linkages.

31. A copolymer (A) according to Claim 30 wherein $R_f'$ is the branched and carbocyclic 4,5-(2,2-trifluoromethyl)-1,3-dioxolanediyl.

32. A copolymer (A) according to Claim 30 wherein $R_f'$ is $-(CF_2CF_2)_n$ and n is an integer from 1 to 6.

33. A copolymer (A) according to Claims 22, 23, or 24 wherein at least one of Z or Z′ is any mesogenic divalent organic radical independently selected.

34. A copolymer (A) according to Claim 33 wherein Z is of the formula

-V-W-V-

wherein v is a divalent radical independently selected from the group consisting of 1,4-phenylene, 4,4,-biphenylene, naphthylidene, 1,4-[2.2.2]-bicyclooctylidene, and 1,4-cyclohexylidene, and W is a divalent radical selected from the group consisting of -CH=N-, -N(O)=N-, -N=N-, -C(=O)-O-, -C≡C-, -CH=CH-, $-CH=C(CH_3)-$, -C(=O)-NH-, -CH=CH-CH=N-, -CH=CH-C(=O)-O-, and -CH=N-N=CH-.

35. A polymer (B) comprising 100 mole percent of at least one independently selected repeating unit of the formula

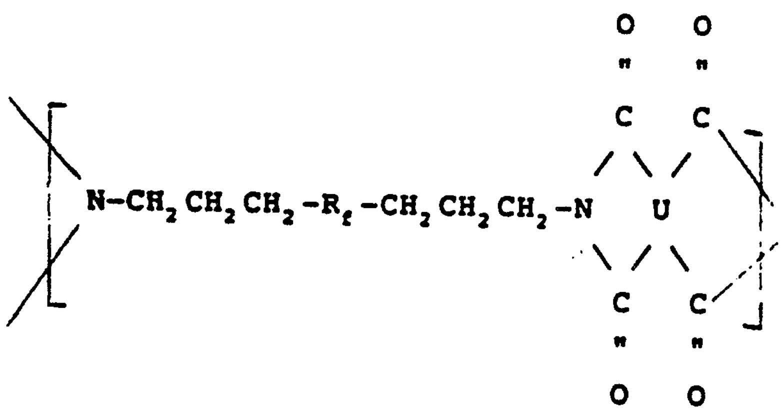 (5)

wherein U is any tetravalent organic radical and $R_f$ is a perfluorinated divalent organic radical selected from the group consisting of linear, branched, carbocyclic, and mixtures thereof having from 1 to about 40 carbon atoms and containing from zero to about 20 ether linkages.

36. A polymer (B) according to Claim 35 wherein $R_f$ is the branched and carbocyclic 4,5-(2,2-trifluoromethyl)-1,3-dioxolanediyl.

37. A polymer (B) according to Claim 35 wherein $R_f$ is $-(CF_2CF_2)_n-$ and n is an integer from 1 to 6.

38. A polymer (B) according to Claims 35, 36, or 37 wherein U is selected from the group consisting of linear, branched, carbocyclic, aromatic, and mixtures thereof having from about 6 to about 20 carbon atoms and containing from 0 to about 5 linkages selected from the group consisting of ether and sulfonyl.

39. A polymer (B) according to Claims 35, 36, or 37 wherein U is selected from the group consisting of 1,2,4,5-benzenetetrayl, 2,2-bis(3,4-phenylene)-propane, and 2,2-bis(3,4-phenylene)-1,1,1,3,3,3-hexafluoropropane.

40. A copolymer (B) comprising from 1 to 99 mole percent of at least one independently selected repeating unit of the formula

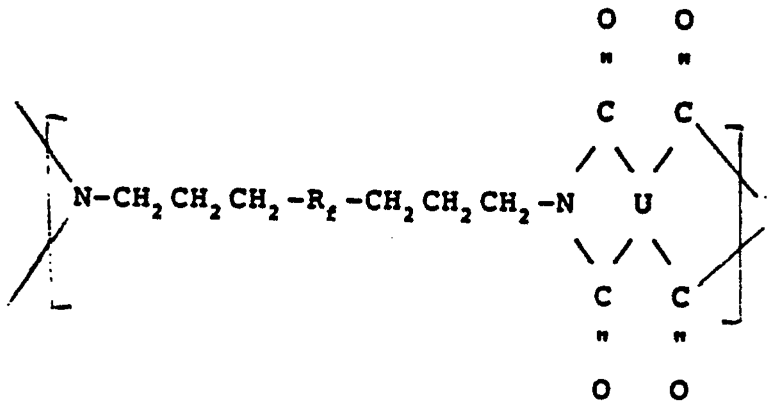

(5)

wherein U is any tetravalent organic radical and $R_f$ is a perfluorinated divalent organic radical selected from the group consisting of linear, branched, carbocyclic, and mixtures thereof having from 1 to about 40 carbon atoms and containing from zero to about 20 ether linkages; and from 99 to 1 mole percent of at least one independently selected repeating unit of the formula

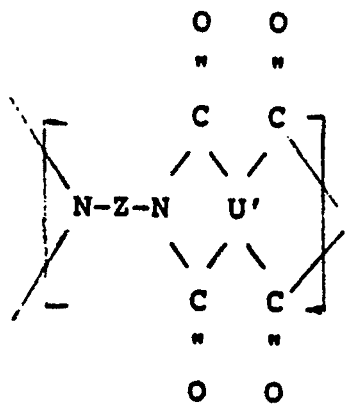

(6)

wherein U′ is any tetravalent organic radical and Z is any divalent organic radical.

41. A copolymer (B) according to Claim 40 wherein $R_f$ is the branched and carbocyclic 4,5-(2,2-trifluoromethyl)-1,3-dioxolanediyl.

42. A copolymer (B) according to Claim 40 wherein $R_f$ is $-(CF_2CF_2)_n-$ and n is an integer from 1 to 6.

43. A copolymer (B) according to Claims 40, 41, or 42 wherein U in repeating unit (5) and U′ in repeating unit (6) are independently selected from the group consisting of linear, branched, carbocyclic, aromatic, and mixtures thereof having from about 6 to about 20 carbon atoms and containing from 0 to about 5 linkages selected from the group consisting of ether and sulfonyl.

44. A copolymer (B) according to Claim 43 wherein Z is selected from the group consisting of linear, branched, carbocyclic, aromatic, and mixtures thereof having from 1 to about 20 carbon atoms and containing from zero to about 10 ether linkages.

45. A copolymer (B) according to Claim 44 wherein Z is selected from the group consisting of 4,4′-diphenylene ether, m-phenylene, and p-phenylene.

46. A copolymer (B) according to Claims 40, 41, or 42 wherein U in repeating unit (5) and U′ in repeating unit (6) are independently selected from the group consisting of 1,2,4,5-benzenetetrayl, 2,2-bis-(3,4-phenylene)propane, and 2,2-bis(3,4-phenylene)-1,1,1,3,3,3-hexafluoropropane.

47. A copolymer (B) according to Claim 46 wherein Z is selected from the group consisting of linear, branched, carbocyclic, aromatic, and mixtures thereof having from 1 to about 20 carbon atoms and containing from zero to about 10 ether linkages.

48. A copolymer (B) according to Claim 47 wherein Z is selected from the group consisting of 4,4′-diphenylene ether, m-phenylene, and p-phenylene.

FIGURE 1